# FASCICULE DE BREVET EUROPEEN

(11) **EP 2 982 977 B1**
(45) Date de publication et mention de la délivrance du brevet: **13.12.2017**
(21) Numéro de dépôt: 15306243.5
(22) Date de dépôt: 30.07.2015
(51) Int. Cl.: G01N 33/00

(54) **DISPOSITIF DE MESURE ET/OU DE DÉTECTION D'UN PRODUIT CONTAMINANT PRÉSENT DANS UN GAZ, NOTAMMENT CENTRALE DE MESURE DE LA QUALITÉ DE L'AIR**
GERÄT ZUR MESSUNG VON VERUNREINIGUNGEN IN EINEM GAS, INSBESONDERE LUFTQUALITÄTS-MESSEINHEIT
MEASUREMENT AND/OR DETECTION DEVICE FOR A CONTAMINATING PRODUCT PRESENT IN A GAS, IN PARTICULAR AIR QUALITY MEASUREMENT UNIT

(30) Priorité: 08.08.2014 FR 1457696
(43) Date de publication de la demande: 10.02.2016
(73) Titulaire: L'AIR LIQUIDE, SOCIETE ANONYME POUR L'ETUDE ET L'EXPLOITATION DES PROCEDES GEORGES CLAUDE, 75007 Paris (FR)
(72) Inventeur: Mignot, Guillaume, 60260 LAMORLAYE (FR); Heron, Christian, 78000 VERSAILLES (FR)
(74) Mandataire: Mellul-Bendelac, Sylvie Lisette

(56) Documents cités:
- US-A- 4 882 576
- US-A1- 2003 216 660
- US-A1- 2013 174 744
- US-A1- 2014 069 281

## Description

L'invention concerne un dispositif de mesure et/ou de détection d'un produit contaminant présent dans un gaz. Bien que non exclusivement, l'invention trouvera avantageusement son application en tant que centrale de mesure de la qualité de l'air.

Il a déjà été proposé des centrales domestiques de mesure de la qualité de l'air. Elles sont destinées à être utilisées par des particuliers ou des collectivités pour contrôler l'atmosphère présente dans les locaux concernés. Elles fonctionnent avec des capteurs donnant une mesure de la concentration du polluant dont on cherche à quantifier la présence. Les concentrations en jeu sont généralement faibles, de l'ordre de quelques centaines à quelques milliers de ppm (parties par million) et les capteurs employés doivent donc être particulièrement sensibles.

Il est connu en ce sens des capteurs utilisant une source émettant un faisceau en direction d'un détecteur. Le faisceau est en partie absorbé par le polluant. La concentration en polluant est ainsi déterminée par comparaison entre une mesure de l'intensité du faisceau en absence de polluant et l'intensité du faisceau partiellement absorbé.

De tels détecteurs connaissent une dérive de leur mesure. Autrement dit, pour une même concentration de polluant, la valeur de la concentration délivrée par le capteur varie d'une mesure à l'autre au fur et à mesure du temps. Dans le domaine des capteurs de mesure de la qualité de l'air, les indications données peuvent varier du simple au double pour une même concentration de polluant. Avec une telle incertitude de mesure, on comprend que l'utilité même de la mesure est compromise.

Pour pallier ce phénomène, il est connu d'étalonner ou de calibrer les capteurs de façon périodique. Pour cela, on met le capteur en contact avec une atmosphère dans laquelle la concentration en polluant est connue et contrôlée et on lance une procédure d'étalonnage ou de calibrage au cours de laquelle on configure le capteur pour qu'il délivre à nouveau une mesure conforme à la concentration en cause.

Dans des domaines industriels ou d'équipements urbains, il est ainsi connu de calibrer des capteurs de mesure de polluant en utilisant des bombonnes de gaz contenant un mélange d'air et d'une quantité, calibrée, de polluant. Les bombonnes sont équipées d'une vanne ou robinet et d'une buse permettant de diriger le gaz en direction des capteurs à tester. Un opérateur transporte la bombonne à proximité du capteur à calibrer, ouvre la vanne ou le robinet de la bombonne, ce qui libère le mélange qui est projeté en direction du capteur à l'aide de la buse. Il referme ensuite la bombonne, ceci tout en lançant une procédure de calibration du capteur. US2003/0216660 A1 donne un autre exemple utilisant des capsules contenant un mélange gazeux pour la calibration d'un capteur de gaz. Le dispositif de mesure permet le logement d'une capsule de calibration par l'intermédiaire d'une cellule à capsule qui se visse sur le dispositif. Une fois la capsule logée, le dispositif peut ensuite percer la capsule afin de diriger le mélange gazeux de calibration vers le capteur à étalonner. On comprend que de telles procédures sont peu pratiques à mettre en oeuvre, notamment compte-tenu du poids de la bombonne. En outre, rien ne limite la quantité de mélange libéré, ce qui peut conduire à certains gaspillages. Cette solution ne convient pas à des utilisations domestiques.

Une autre solution pour étalonner les capteurs de centrales de mesure de la qualité de l'air, en particulier des centrales domestiques, serait de pouvoir compter sur la présence d'une atmosphère ambiante de référence à intervalles réguliers. Par exemple, dans l'hypothèse où, en pleine nuit, la pollution atmosphérique retomberait toujours à un même niveau, on pourrait lancer une étape de calibration de la centrale de mesure à un moment choisi de la nuit. Malheureusement, cette hypothèse est fausse, surtout en milieu urbain où la qualité de l'air est susceptible de varier fortement, même d'une nuit sur l'autre.

L'invention a pour but de pallier les inconvénients précités et propose à cette fin un dispositif de mesure et/ou de détection d'un produit contaminant présent dans un gaz, notamment une centrale de mesure de la qualité de l'air, ledit dispositif de mesure comprenant au moins un capteur de mesure et/ou de détection dudit produit contaminant, ledit dispositif comprenant en outre des moyens de vidange d'une capsule contenant un mélange gazeux de calibration, lesdits moyens de vidange étant configurés pour mettre ledit capteur en présence dudit mélange gazeux, ledit dispositif étant configuré pour calibrer ledit capteur.

Grâce à la calibration, on fiabilise les mesures délivrées par le capteur équipant le dispositif conforme à l'invention. En outre, grâce aux moyens de vidange prévus sur ledit dispositif pour alimenter le capteur à étalonner, le processus de calibration s'effectue de façon maîtrisée, sans gaspillage. L'utilisation de l'invention sous forme de centrale domestiques est favorisée car seules des manipulations simples sont nécessaires pour sa mise en oeuvre.

Selon différentes caractéristiques de l'invention qui pourront être prises ensemble ou séparément :
- Lesdits moyens de vidange comprennent un logement destiné à accueillir ladite capsule,
- Ledit logement comprend un fond et une paroi latérale, débouchant sur une ouverture d'introduction de la capsule dans ledit logement,
- Lesdits moyens de vidange comprennent en outre un plateau, mobile dans ledit logement, et des moyens de rappel, configurés pour déplacer ledit plateau dans ledit logement,
- Les moyens de rappel comprennent un ressort,
- Ledit ressort est positionné entre le fond dudit logement et ledit plateau,
- Ledit plateau comprend un joint d'étanchéité, destiné à coopérer avec ladite capsule, lorsque ladite capsule est positionnée dans ledit logement,
- Lesdits moyens de vidange comprennent en outre un conduit de vidange,
- Ledit logement est configuré pour positionner, en particulier centrer, ladite capsule par rapport audit conduit de vidange, de sorte que celui-ci se trouve en vis-à-vis d'une zone choisie de la capsule,
- Ledit conduit de vidange comprend au niveau de son extrémité distale une connectique configuré pour percer ladite capsule
- Ladite connectique est en forme d'hameçon,
- Ladite connectique est en outre configurée pour s'arrimer à l'intérieur de la capsule,
- Ladite connectique présente une forme en pointe munie d'un bord postérieur et d'un bord antérieur, un canal du conduit de vidange débouchant au niveau dudit bord postérieur et ledit bord antérieur étant configuré pour prendre appui contre une face intérieure de la capsule,
- Ledit dispositif comprend une chambre d'accumulation au voisinage de laquelle se trouve ledit capteur de sorte qu'il puisse mesurer et/ou détecter ledit produit contaminant présent dans le gaz se trouvant dans ladite chambre d'accumulation,
- Ledit conduit de vidange débouche à son extrémité proximale au niveau de ladite chambre d'accumulation,
- Ledit capteur est un capteur de concentration du produit contaminant dans ledit gaz,
- Ledit dispositif comprend en outre un capteur de détection de ladite capsule, configuré pour délivré une information de présence lorsque ladite capsule est introduite dans ledit logement,
- Ledit dispositif comprend des moyens de traitement, configurés pour exécuter une procédure de calibration après réception de ladite information de présence.

L'invention concerne aussi un ensemble d'un dispositif de mesure et/ou de détection tel que décrit plus et au moins un capsule, destinée à l'étalonnage dudit dispositif, ladite capsule étant avantageusement prévue à usage unique.

Autrement dit, ladite capsule contient un gaz, dit principal, et un produit contaminant, formant ledit mélange gazeux de calibration, ladite capsule étant configurée pour contenir ledit mélange sous pression et forcer une libération de l'intégralité dudit mélange en une seule dose.

Encore autrement dit, selon ce mode de mise en oeuvre, l'invention exclue qu'une vidange seulement partielle de la capsule puisse avoir lieu. On évite de la sorte la manipulation de capsules à ouvrir et fermer entre chaque usage, ce qui renforce encore le caractère pratique d'utilisation de l'invention.

Ledit gaz principal est, par exemple, un mélange de composés gazeux, en particulier de l'air.

Ledit produit contaminant est choisi, par exemple, parmi les polluants atmosphériques, notamment, le dioxyde de carbone, le monoxyde de carbone, un oxyde d'azote, un oxyde de souffre, un ou des composés organiques volatiles, des particules, en particulier de la fumée issue d'une combustion, ou un mélange de ces composés.

Selon différentes caractéristiques de cet aspect de l'invention, qui pourront être prise ensemble ou séparément :
- la concentration du produit contaminant dans le gaz principal est comprise entre 1, notamment 100, et 10.000 ppm,
- la pression du mélange dans la capsule est inférieure à 20 bars, voire à 10 bars, voire à 5 bars,
- le volume de la capsule est inférieur à 200 cm3, voire à 100 cm3, voire à 50 cm3, voire 25 cm3
- ladite capsule est configurée pour permettre le dégagement d'une ouverture de libération dudit mélange par perçage,
- ladite capsule comprend un orifice de libération dudit mélange et un obturateur dudit orifice,
- ledit obturateur est configuré pour être percé,
- ledit obturateur est une membrane,
- ladite capsule est configurée pour permettre une vidange par retrait dudit obturateur
- ledit obturateur est un bouchon,
- ledit obturateur est configuré pour être escamoté.

D'autres caractéristiques et avantages de l'invention apparaîtront à la lecture de la description détaillée qui suit pour la compréhension de laquelle on se reportera aux dessins annexés dans lesquels :
- la figure 1 est une vue en coupe longitudinale illustrant schématiquement une partie d'un dispositif de mesure et/ou de détection selon l'invention et d'une capsule destiné à être utilisé avec ledit dispositif,
- la figures 2 est une vue similaire à la figure 2 illustrant la mise en place de la capsule sur ledit dispositif,
- la figure 3 est une vue similaire à la figure 2 illustrant la capsule en place sur le dispositif lors de la libération du gaz de calibration qu'elle contient,
- la figure 4 est une vue similaire à la figure 2 illustrant le retrait de la capsule vide dudit dispositif,
- la figure 5 est une vue perspective de la capsule des figures précédentes,
- la figure 6 est une vue en perspective illustrant une variante de réalisation de la capsule de la figure 5,
- la figure 7 est une vue en perspective d'un exemple de dispositif de mesure et/ou de détection conforme à l'invention, en condition normale d'utilisation,
- la figure 8 est une vue en perspective du dispositif de la figure 7, prête à être étalonnée.

Dans la description qui va suivre, des référence identiques désignent des pièces identiques ou ayant des fonctions similaires.

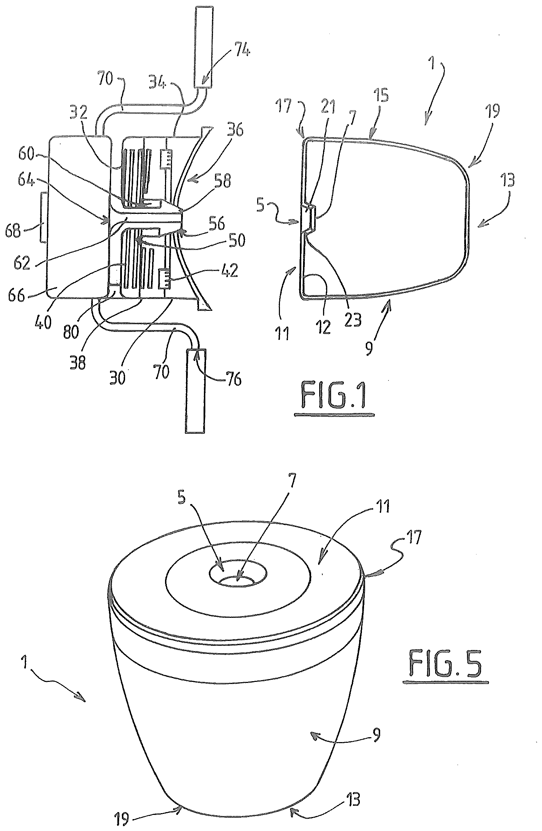

Comme illustré aux figures 1 à 4, l'invention concerne un dispositif de mesure et/ou de détection d'un produit contaminant présent dans un gaz. Il pourra notamment s'agir d'une centrale de mesure de la qualité de l'air, en particulier d'une centrale domestique. Ledit dispositif de mesure comprend au moins un capteur 68 de mesure et/ou de détection dudit produit contaminant.

Selon l'invention, ledit dispositif est configuré pour calibrer ledit capteur 68. Ledit dispositif comprend en outre des moyens de vidange d'une capsule 1 contenant un mélange gazeux de calibration et ledit dispositif est configuré pour mettre ledit capteur 68 en présence dudit mélange gazeux, issu de la capsule 1, par l'intermédiaire desdits moyens de vidange.

Lesdits moyens de vidange comprennent ici un logement 30 destiné à accueillir ladite capsule 1, en particulier au niveau d'un couvercle 11 de ladite capsule. Ledit logement est, par exemple, de forme sensiblement cylindrique. Ledit logement 30 comprend un fond 32 et une paroi latérale 34, débouchant sur une ouverture 36 d'introduction de la capsule dans ledit logement 30, située ici à l'opposée dudit fond 32 du logement 30.

Ledit dispositif peut aussi comprendre par exemple en outre un plateau 38, mobile dans ledit logement 30, et un ressort 40, positionné entre le fond 32 dudit logement 30 et ledit plateau 38. Ledit plateau 38 est ici équipé d'un joint d'étanchéité 42, annulaire, destiné à coopérer avec ladite capsule 1, en particulier son couvercle 11, lorsque ladite capsule 1 est positionnée dans ledit logement 30. Cela étant, un tel plateau mobile n'est pas nécessaire et le logement 30 pourra, en variante, être configuré pour que la capsule vienne directement en prise sur le fond de celui-ci, notamment en prévoyant le joint d'étanchéité au niveau dudit fond.

Ledit dispositif comprend en outre un conduit de vidange 50. Ledit conduit 50 passe à travers ledit fond 32 du logement 30 et à travers ledit plateau 38, par des lumières prévues dans ceux-ci, notamment en leur centre.

On comprend que ledit logement 30 est configuré pour positionner, en particulier centrer, ladite capsule par rapport audit conduit de vidange 50, de sorte que celui-ci se trouve en vis-à-vis d'une zone de capsule 1 à percer, en particulier un orifice de libération 5 de ladite capsule.

Ledit conduit de vidange 50 comprend au niveau de son extrémité distale une connectique 56 en forme d'hameçon, configuré pour percer ledit orifice de libération 5 et s'arrimer à l'intérieur de la capsule contre une face intérieure 12 du couvercle 11. Plus précisément, ici, ladite connectique 56 présente une forme en pointe munie d'un bord postérieur 58 et d'un bord antérieur 60. Un canal 62 du conduit de vidange 50 débouche au niveau dudit bord postérieur 58. Ledit bord antérieur 60 est configuré pour prendre appui contre ladite face intérieure 12 du couvercle, notamment contre l'extrémité libre d'un bord plié 23 dudit couvercle 11.

Ledit conduit de vidange 50 débouche ici à son extrémité proximale 64 au niveau d'une chambre 66 d'accumulation du gaz. Le capteur 68 est situé en regard de ladite chambre 66. Il s'agit, par exemple, d'un capteur permettant de mesurer la concentration du produit polluant en cause. Ladite chambre 66 est reliée à l'extérieur, ici par des canaux 70, 72 débouchant par des évents 74, 76.

Ledit mélange gazeux de calibration, contenu dans ladite capsule, comprend le produit contaminant et le gaz, dit principal, en cause. Ledit gaz principal est, par exemple, un mélange de composés gazeux, notamment de l'air. Ledit produit contaminant est choisi, par exemple, parmi les polluants atmosphériques tels que, le dioxyde de carbone, le monoxyde de carbone, un oxyde d'azote, un oxyde de souffre, un ou des composés organiques volatiles, des particules, en particulier de la fumée issue d'une combustion, ou un mélange de ces composés.

Ladite capsule est configurée pour contenir ledit mélange sous pression. On entend par là que ledit mélange est à une pression supérieure à la pression atmosphérique dans ladite capsule de sorte qu'à l'ouverture de ladite capsule, il s'écoulera naturellement vers l'extérieur. Cela étant, une pression excessive n'est pas non plus nécessaire. La pression du mélange dans la capsule pourra ainsi être inférieure à 20 bars, voire à 10 bars, voire à 5 bars. De tels niveaux de pression permettent d'utiliser des capsules dont les coûts de fabrication sont limités.

De façon préférentielle, ladite capsule est configurée pour forcer une libération de l'intégralité dudit mélange en une seule dose. Autrement dit, un fois la capsule ouverte, elle ne peut plus être refermée tant que la dose de mélange qu'elle contient ne s'est pas entièrement échappée. En particulier, ladite capsule ne comprend pas de mécanisme d'ouverture/fermeture, tels que des vannes ou robinets, permettant de la refermer alors que l'intégralité du mélange ne s'est pas encore évacué. Encore autrement dit, ladite capsule est à usage unique.

Elle est de la sorte pratique à manier et à utiliser, notamment en raison de son volume réduit comparé aux bombonnes de gaz connues. A ce sujet, le volume de la capsule est avantageusement inférieur à 200 cm3, voire à 100 cm3, voire à 50 cm3, voire à 25 cm3.

La concentration du produit contaminant dans le gaz principal est comprise, par exemple, 1 et 10.000 ppm, notamment entre 100 et 10.000 ppm. Pour du dioxyde de carbone, elle pourra être, notamment, d'environ 500 ppm.

Comme illustré à la figure 5, ladite capsule 1 est avantageusement configurée pour permettre le dégagement d'une ouverture de libération dudit mélange par perçage. Ladite capsule 1 comprend ici pour cela l'orifice de libération 5 dudit mélange et un obturateur 7 dudit orifice, ledit obturateur 7 étant configuré pour être percé. Il s'agit, par exemple, d'une membrane.

La capsule 1 comprend, par exemple, un corps 9 et le couvercle 11, fermant ici ledit corps 1. Comme déjà indiqué, c'est avantageusement au niveau du couvercle 11 que le dégagement de l'ouverture de libération du mélange a lieu. Autrement dit, ledit orifice de libération 5 du mélange est situé au niveau dudit couvercle 11, par exemple en son centre.

Selon une première variante, ledit corps 9 et/ou ledit couvercle 11 sont formés par emboutissage, le couvercle 11 étant rapporté sur ledit corps 9, par exemple par sertissage. Selon une autre variante, ledit corps 9 et ledit couvercle 11 sont issus de matière l'un de l'autre. Ils pourront alors être formés par soufflage.

Ledit corps 9 et/ou ledit couvercle 11 sont, notamment, à base d'aluminium. Ils pourront présenter une épaisseur de matière comprise entre 0.1 et 1 mm. Ils pourront aussi être en matériau plastique, notamment en PVC.

Ladite capsule 1, plus particulièrement ledit corps 9, comprend avantageusement un fond 13 plat. Une telle forme facilite le stockage de la capsule et la manipulation de la capsule, notamment lors de son remplissage.

Ladite capsule comprend en outre ici une paroi latérale 15 reliant ledit couvercle 11 et ledit fond 13. Ladite paroi latérale 15 est avantageusement de section arrondie. Ladite capsule pourra en outre présenter des rayons 17, 19 reliant ladite paroi latérale 15 et ledit couvercle 11 et/ou ledit ladite paroi latérale 15 et ledit fond 13. Une telle configuration, en évitant la présence d'arrête vive, facilite la tenue à la pression de la capsule 1.

Ledit couvercle 11 comprend ici un canal 21 de passage du mélange contenu dans la capsule. Ledit canal 21 est formé, par exemple, par le bord plié 23 du couvercle 11, ici tourné vers l'intérieur de la capsule. Ladite membrane 7 est fixée au niveau du l'extrémité libre dudit bord plié 23. Plus largement, ladite membrane 7 pourra être fixée à l'extrémité du canal 21 située en regard de l'intérieur de capsule. Ladite membrane 7 est de la sorte située en retrait et protégée des agressions extérieures. Ledit couvercle 11 est avantageusement plat, ce qui favorisera un arrimage des moyens de vidange de la capsule, en particulier un arrimage étanche. On évite de la sorte les fuites de mélange dans des directions non désirées à l'ouverture de la capsule.

Si l'on se reporte de nouveau à la figure 1, on constate que celle-ci illustre plus particulièrement la situation avant mise en place de la capsule 1 dans le logement 30. Le ressort 40 est alors au repos et la connectique 56 est située par exemple juste au-delà du plateau mobile 38, vers l'extérieur.

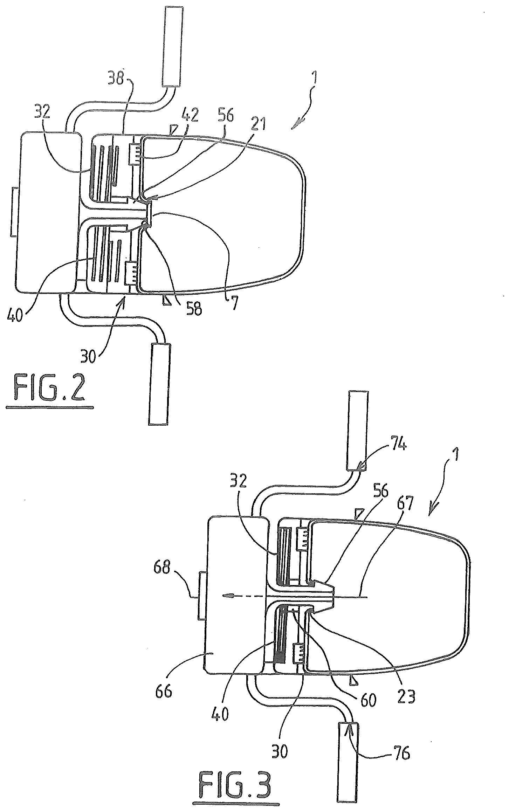

La figure 2 illustre la situation lors de l'introduction de la capsule 1 dans le logement 30. La capsule 1 vient alors, par exemple, en contact avec le plateau mobile 38. Le bord postérieur 58 de la connectique 56 est à ce moment là à l'intérieur du canal de passage 21 de la capsule mais sans avoir encore perforé la membrane 7. La raideur du ressort 40 est avantageusement choisie pour que le joint d'étanchéité 42 soit comprimé par la capsule avant que le plateau mobile ne se déplace vers le fond 32 dudit logement.

La figure 3 illustre la situation après avoir poussé la capsule 1 en direction du fond 32 du logement 30 en lutant contre la force du ressort 40. La connectique 56 a alors percé la membrane 7 et se trouve insérée dans la capsule 1 dans laquelle elle est maintenue par le contact établi entre son bord antérieur 60 et l'extrémité libre du bord plié 23 de ladite capsule 1. La vidange du mélange contenu dans la capsule 1 en direction de ladite chambre d'accumulation 66 a alors lieu, selon la flèche repérée 67, ceci sous l'effet de la différence de pression entre l'intérieur de la capsule et la pression dans ladite chambre d'accumulation 66, qui se trouve à la pression atmosphérique par l'intermédiaire des évents 74, 76. On met de la sorte le capteur 68 en présence d'un mélange dans lequel la quantité de produit contaminant est connu.

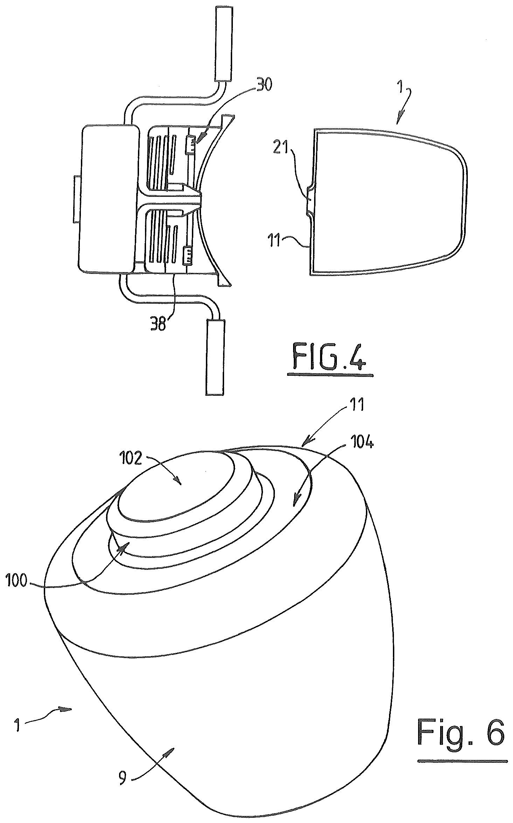

La figure 4 illustre le retrait de la capsule hors du logement 30. Un tel retrait pourra avoir lieu par arrachage de la capsule, l'épaisseur de matière du couvercle 11 étant choisie en ce sens afin de permettre un retournement du canal de passage 21 de ladite capsule. A titre d'exemple, de son côté, le plateau mobile 38 retrouve sa position initiale.

Le remplissage de la capsule 1 pourra avoir eu lieu en usine, par exemple à partir de bouteilles de grandes contenances préparées par voie gravimétrique. On dispose de la sorte dans la capsule d'un produit contaminant dosé de façon très précise car résultant du dosage fait en usine pour le remplissage de la bouteille de grande contenance.

Comme illustré à la figure 6, selon une variante de réalisation de la capsule, ledit couvercle 11 comprend un col 100 entre une surface d'interface 102 et une partie 104 de section élargie. Ladite surface d'interface 102 est, par exemple, ici la partie du couvercle destinée à être introduite dans ledit logement 30 pour coopérer avec ledit plateau mobile 38. En diminuant de la sorte la taille de la partie de la capsule destiné à coopérer avec le dispositif de vidange, on rend plus facile l'utilisation d'une même capsule sur un grand nombre de dispositifs de configuration différente. Ladite surface d'interface 102 pourra présenter un diamètre inférieur à 30 mm, notamment inférieur à 25 mm.

Ladite surface d'interface 102 est configurée pour permettre le dégagement de ladite ouverture de libération du mélange par perçage. Elle pourra en particulier être munie dudit orifice de libération du mélange, non illustré à cette figure. Ladite surface d'interface est avantageusement plane.

Selon un autre mode de réalisation de l'obturateur de la capsule, non illustré, ce dernier pourra être configuré pour être escamoté à l'intérieur de la capsule. Il s'agit, par exemple, d'un bouchon.

Selon cet autre mode de réalisation, ledit obturateur est avantageusement configuré pour être repositionné dans ledit orifice. Plus précisément, ledit bouchon pourra être muni d'une tige. Lorsque ledit bouchon obture ledit orifice de libération du mélange, la tige est configurée pour se trouver intégralement en dehors de la capsule. Lorsque le bouchon a été escamoté à l'intérieur de la capsule, la tige est configurée pour rester au moins partiellement en dehors de la capsule. Son extrémité située à l'extérieur peut de la sorte être manipulée pour repositionner le bouchon à l'intérieur dudit orifice de réception, en tirant sur la tige.

Une telle configuration permet de pouvoir facilement fermer la capsule après un remplissage initial de la capsule.

Elle permet également de pouvoir remplir à nouveau la capsule, après chaque vidange. Cette possibilité technique est permise par cette configuration de fermeture de la capsule, même si on l'a dit plus haut l'esprit de la présente invention est de proposer une capsule à usage unique.

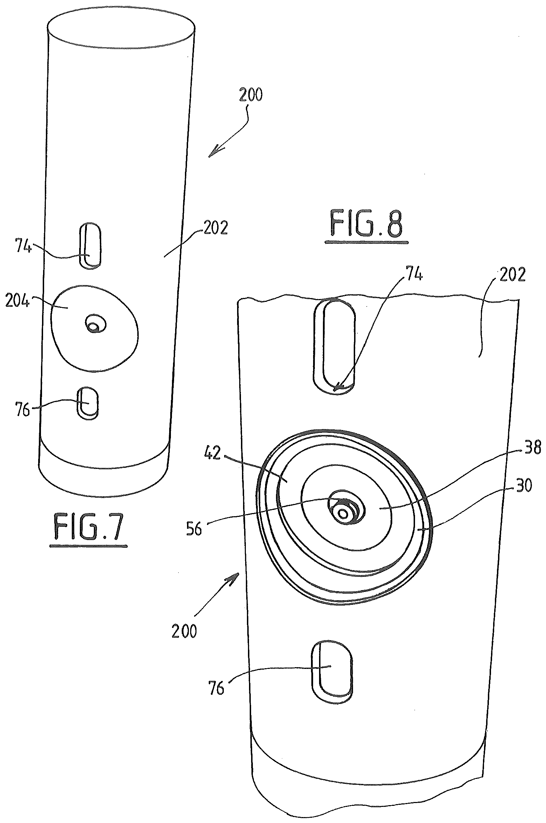

A la figure 7, une centrale domestique de mesure de la qualité de l'air 200 destinée à être étalonnée à l'aide de ladite capsule 1 a été illustrée, dans son état de fonctionnement normal, c'est-à-dire, en dehors d'une phase d'étalonnage.

Ladite centrale 200 comprend une chambre d'accumulation de l'air telle que la chambre d'accumulation 66 déjà évoquée, non visible à cette figure. Les évents 74, 76 sont ici prévus au niveau d'un capot 202 de ladite centrale. Ledit capot 202 est, par exemple, de forme sensiblement cylindrique.

Ladite centrale 200 pourra encore comprendre différents organes de traitement, non illustrés, permettant à la centrale d'afficher et/ou de transmettre à distance le résultat de la mesure.

Ledit capot 202 présente ici une forme sensiblement cylindrique définissant une cavité dissimulant ledit capteur, ladite chambre d'accumulation et/ou lesdits moyens de traitement.

A la figure 8, ladite centrale 200 a été préparée pour une phase d'étalonnage. Pour cela une trappe amovible 204 de ladite centrale 200 a été retirée. Elle donne accès à un logement 30 tel que celui des moyens de vidange décrits plus haut, ledit logement 30 étant ici intégré dans le capot 204. On distingue le plateau mobile 38 et son joint d'étanchéité 42 ainsi que la connectique 56, le reste du dispositif de vidange se trouvant sous le capot 202.

De manière à autoriser la calibration du capteur, lesdits organes de traitement de la centrale sont avantageusement configurés pour permettre une phase d'étalonnage de la mesure. Pour cela ladite centrale pourra être munie d'un capteur de détection de la présence d'une capsule 1 dans le logement 30, illustré de façon schématique 80 aux figures 1 à 4. Ladite phase d'étalonnage est ainsi lancée après que ledit capteur ait délivré une information de présence auxdits organes de traitement. On s'assure de la sorte de la présence de la capsule et donc de la présence du mélange gazeux à proximité du capteur à étalonner, suite à la vidange de ladite capsule par l'intermédiaire des moyens de vidange. En variante, la phase d'étalonnage pourra être déclenchée par un utilisateur, par exemple par actionnement d'un bouton prévu sur la centrale ou une commande à distance, les moyens de traitement étant alors configurés pour traiter l'actionnement du bouton comme un signal d'ordre d'exécution de ladite phase d'étalonnage.

Cela étant, l'invention pourra trouver de nombreuses autres applications. Tout d'abord, ledit dispositif de mesure et/ou de détection pourra servir à la mesure et/ou à la détection de plusieurs produits contaminants. Son étalonnage pourra alors se faire à l'aide de la même capsule, contenant chacun des produits contaminant en cause, en quantité contrôlé. Ledit dispositif de mesure et/ou de détection pourra aussi contenir plusieurs capteurs.

Par ailleurs, ladite capsule pourra servir à l'étalonnage de capteur de concentration se trouvant dans tout type de dispositif sans qu'il s'agisse nécessairement de centrales de contrôle de la qualité de l'air, en particulier de centrales domestiques. Il pourra s'agir, par exemple, d'extracteurs, de climatiseurs et/ou de détecteurs de fumée munis de capteurs mesurant la concentration d'un produit contaminant dans l'air ambiant.

Dans de tels cas, le résultat de la mesure effectué par le capteur à calibrer a pour vocation de permettre de contrôler lesdits appareils, notamment en relation avec un seuil de déclenchement.

## Revendications

1. Dispositif de mesure et/ou de détection d'un produit contaminant présent dans un gaz, notamment centrale de mesure de la qualité de l'air, ledit dispositif de mesure comprenant au moins un capteur (68) de mesure et/ou de détection dudit produit contaminant, ledit dispositif comprenant en outre des moyens de vidange d'une capsule (1) contenant un mélange gazeux de calibration, lesdits moyens de vidange étant configurés pour mettre ledit capteur (68) en présence dudit mélange gazeux, ledit dispositif étant configuré pour calibrer ledit capteur (68),
- lesdits moyens de vidange comprenant un logement (30) destiné à accueillir ladite capsule (1),
- lesdits moyens de vidange comprenant en outre un conduit de vidange (50),
- ledit logement (30) étant configuré pour positionner ladite capsule (1) par rapport audit conduit de vidange (50), de sorte que celui-ci se trouve en vis-à-vis d'une zone choisie de la capsule (1), **se caractérisant en ce que**
- ledit conduit de vidange (50) comprend au niveau de son extrémité distale une connectique (56) en forme d'hameçon, configurée pour percer ladite capsule (1), et configurée pour s'arrimer à l'intérieur de la capsule (1).

2. Dispositif selon la revendication 1, dans lequel ladite connectique (56) présente une forme en pointe munie d'un bord postérieur (58) et d'un bord antérieur (60).

3. Dispositif selon la revendication précédente dans lequel un canal (62) du conduit de vidange (50) débouche au niveau dudit bord postérieur (58) et ledit bord antérieur (60) est configuré pour prendre appui contre une face intérieure (12) de la capsule (1).

4. Dispositif selon l'une quelconque des revendications 1 à 3, comprenant en outre une chambre (66) d'accumulation au voisinage de laquelle se trouve ledit capteur (68) de sorte qu'il puisse mesurer et/ou détecter ledit produit contaminant présent dans le gaz se trouvant dans ladite chambre d'accumulation, ledit conduit de vidange (50) débouchant à son extrémité proximale (64) au niveau de ladite chambre (66) d'accumulation.

5. Dispositif selon l'une quelconque des revendications précédentes dans lequel ledit dispositif comprend un capteur de détection (80) de ladite capsule (1), configuré pour délivré une information de présence lorsque ladite capsule est introduite dans ledit logement.

6. Dispositif selon la revendication précédente dans lequel ledit dispositif comprend des moyens de traitement, configurés pour exécuter une procédure de calibration après réception de ladite information de présence.

7. Dispositif selon l'une quelconque des revendications précédentes dans lequel ledit capteur (68) est un capteur de concentration du produit contaminant dans ledit gaz.

8. Ensemble d'un dispositif de mesure et/ou de détection selon l'une quelconque des revendications précédentes et d'au moins un capsule (1) destinée à l'étalonnage dudit dispositif, ladite capsule (1) étant prévue à usage unique, par le fait qu'elle est configurée pour contenir ledit mélange sous pression et forcer une libération de l'intégralité dudit mélange en une seule dose lors de ladite vidange.

## Patentansprüche

1. Vorrichtung zum Messen und/oder Erkennen eines Kontaminanten, der in einem Gas präsent ist, insbesondere Luftqualitäts-Messeinheit, wobei die Messvorrichtung mindestens einen Sensor (68) zum Messen und/oder Erkennen des Kontaminanten umfasst, wobei die Vorrichtung weiter Mittel zum Entleeren einer Kapsel (1) umfasst, die ein Kalibrier-Gasgemisch enthält, wobei die Entleermittel dafür eingerichtet sind, den Sensor (68) in Präsenz des Gasgemisches zu bringen, wobei die Vorrichtung dafür eingerichtet ist, den Sensor (68) zu kalibrieren,
wobei die Entleermittel eine Aufnahme (30) umfassen, die dazu bestimmt ist, die Kapsel (1) aufzunehmen,
wobei die Entleermittel weiter eine Entleerleitung (50) umfassen,
wobei die Aufnahme (30) dafür eingerichtet ist, die Kapsel (1) in Bezug auf die Entleerleitung (50) derart zu positionieren, dass sich diese einem ausgewählten Bereich der Kapsel (1) gegenüber befindet, **dadurch gekennzeichnet, dass**
die Entleerleitung (50) auf Höhe ihres distalen Endes einen hakenförmigen Verbinder (56) umfasst, der dafür eingerichtet ist, die Kapsel (1) zu durchstechen, und dafür eingerichtet ist, sich im Inneren der Kapsel (1) zu verankern.

2. Vorrichtung nach Anspruch 1, wobei der Verbinder (56) eine spitze Form aufweist, die mit einer hinteren Kante (58) und mit einer vorderen Kante (60) versehen ist.

3. Vorrichtung nach dem vorstehenden Anspruch, wobei ein Kanal (62) der Entleerleitung (50) auf Höhe der hinteren Kante (58) mündet und die vordere Kante (60) dafür eingerichtet ist, gegen eine Innenseite (12) der Kapsel (1) anzuliegen.

4. Vorrichtung nach einem der Ansprüche 1 bis 3, weiter eine Sammelkammer (66) umfassend, in deren Nachbarschaft sich der Sensor (68) befindet, derart, dass er den Kontaminanten, der in dem in der Sammelkammer befindlichen Gas präsent ist, messen und/oder erkennen kann, wobei die Entleerleitung (50) an ihrem proximalen Ende (64) auf Höhe der Sammelkammer (66) mündet.

5. Vorrichtung nach einem der vorstehenden Ansprüche, wobei die Vorrichtung einen Erkennungssensor (80) der Kapsel (1) umfasst, der dafür eingerichtet ist, eine Präsenzinformation auszugeben, wenn die Kapsel in die Aufnahme eingesetzt wird.

6. Vorrichtung nach dem vorstehenden Anspruch, wobei die Vorrichtung Verarbeitungsmittel umfasst, die dafür eingerichtet sind, nach Empfang der Präsenzinformation einen Kalibriervorgang auszuführen.

7. Vorrichtung nach einem der vorstehenden Ansprüche, wobei der Sensor (68) ein Konzentrationssensor des Kontaminanten in dem Gas ist.

8. Anordnung aus einer Mess- und/oder Erkennungsvorrichtung nach einem der vorstehenden Ansprüche und mindestens einer Kapsel (1), die zum Eichen der Vorrichtung bestimmt ist, wobei die Kapsel (1) zur einmaligen Verwendung vorgesehen ist durch die Tatsache, dass sie dafür eingerichtet ist, das Gemisch unter Druck zu enthalten und bei der Entleerung eine Freisetzung der Gesamtheit des Gemisches in einer einzelnen Dosis zu erzwingen.

## Claims

1. Device for measuring and/or detecting a contaminant product in a gas, in particular air quality measuring station, said measuring device comprising at least one sensor (68) for measuring and/or detecting said contaminant product, said device further comprising means for draining a capsule (1) containing a gaseous calibration mixture, said draining means being configured to put said sensor (68) in the presence of said gaseous mixture, said device being configured to calibrate said sensor (68),
said draining means comprising a housing (30) intended to hold said capsule (1),
said draining means further comprising a draining duct (50),
said housing (30) being configured to position said capsule (1) in relation to said draining duct (50), so that it is located opposite a chosen zone of the capsule (1), being **characterised in that**
said draining duct (50) comprises at the level of its distal end, an electrical connector (56) in the form of a hook, configured to pierce said capsule (1), and configured to be stowed inside the capsule (1).

2. Device according to claim 1, wherein said electrical connector (56) has a tip form, equipped with a back edge (58) and a front edge (60).

3. Device according to the preceding claim, wherein a channel (62) of the draining duct (50) leads to the level of said back edge (58) and said front edge (60) is configured to support against an interior face (12) of the capsule (1).

4. Device according to any one of the claims 1 to 3, further comprising a storage chamber (66) near which said sensor (68) is located, so that it can measure and/or detect said contaminant product present in the gas being located in said storage chamber, said draining duct (50) leading to its proximal end (64) at the level of said storage chamber (66).

5. Device according to any one of the preceding claims, wherein said device comprises a sensor for detecting (80) said capsule (1), configured to deliver presence information when said capsule is entered into said housing.

6. Device according to the preceding claim, wherein said device comprises processing means, configured to execute a calibration procedure after receiving said presence information.

7. Device according to any one of the preceding claims, wherein said sensor (68) is a sensor to sense the concentration of the contaminant product in said gas.

8. Measuring and/or detection device unit according to any one of the preceding claims and of at least one capsule (1) intended to calibrate said device, said capsule (1) being provided for single use, by the fact that it is configured to contain said mixture under pressure and force a release of all said mixture in one single dose during said draining.
